# EUROPEAN PATENT APPLICATION

(11) **EP 1 464 274 A1**
(43) Date of publication of application: **06.10.2004**
(21) Application number: 03252141.1
(22) Date of filing: 04.04.2003
(51) Int. Cl.: A61B 5/022

(54) **Inflation bulb for sphygmomanometer**

(71) Applicant: Rossmax International LTD., Taipei, 114 (TW)
(72) Inventor: Kiao, Yu-chi, Kuang-Fu S Rd., Taipei (TW); Chiang, Yi-Sheng, Chi-Lung City, Taipei (TW)
(74) Representative: Johnson, Terence Leslie

(57) **Abstract**

An inflation bulb (200), suitable for use with a sphygmomanometer, is internally hollow, and comprises a gas inlet end (210) and a gas outlet end (220). An inner side of the gas inlet end (210) forms inwardly protruding lips (212) that are approximately flat. The lips (212) define a gas inlet opening (213) through which exterior air gas is allowed to penetrate the inflation bulb (200). The gas outlet end (220) includes a gas outlet opening (222). The gas outlet opening (222) is further connected to a gas intake pipe (210). When the inflation bulb (200) is pressed, the lips (212) occlude the gas inlet opening (210), which compels an inner air gas to exit via the gas outlet opening (222). Once the pressing action is released, the inflation bulb (200) recovers its initial state and causes the lips (212) to open the gas inlet opening (213). By repeatedly pressing and releasing the inflation bulb (200), a cuff of the sphygmomanometer is inflated.

## Description

The invention relates generally to an inflation bulb for sphygmomanometer and, more specifically, to an inflation bulb that may be formed in a single bodv without the need of additional valve assembly.

Current sphygmomanometers are usually distinguished in different types including automatic electric sphygmomanometers, semi-automatic electric sphygmomanometers, and mercury type sphygmomanometers. The semi-automatic electric sphygmomanometer and the mercury type sphygmomanometer conventionally need the use of an inflation bulb. By repeated press and release of the inflation bulb, air gas is manually delivered to inflate the cuff of the sphygmomanometer.

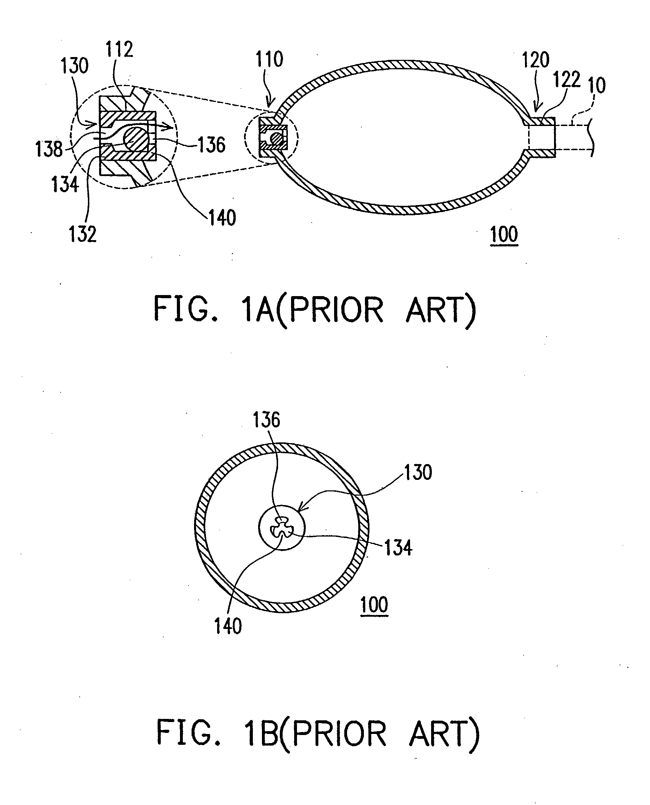

FIG. 1A and FIG. 1B are respectively longitudinal and transversal views particularly illustrating an inflation bulb for sphygmomanometer known in the prior art. The inflation bulb 100 is internally hollow, and terminates in a gas inlet end 110 and a gas outlet end 120. The gas inlet end 110 is provided with a gas inlet opening 112 that includes a unidirectional gas valve 130. The gas outlet end 120 is further provided with a gas outlet opening 122 connected to a gas intake pipe 10. Conventionally, the gas intake pipe 10 further connects to a gas reverse-flow preventing device (not shown). The unidirectional gas valve 130 is constructed from a main body 132, the hollow interior of which mounts a sealing ball 134. Two opposite ends of the valve 130 are respectively provided with through holes 136, 138. The through hole 136 has a diameter larger than that of the sealing ball, and is provided with abutment fingers 140 that protrude at spaced intervals from one another at an edge of the through hole 136 to limit the move of the sealing ball within the main body 132. The through hole 138 has a diameter smaller than that of the sealing ball 134.

When the inflation bulb 100 is not pressed and is in its initial state, exterior air penetrates the inflation bulb 100 via the communicating hole 136 of the valve 130, having a diameter larger than that of the sealing ball 134.

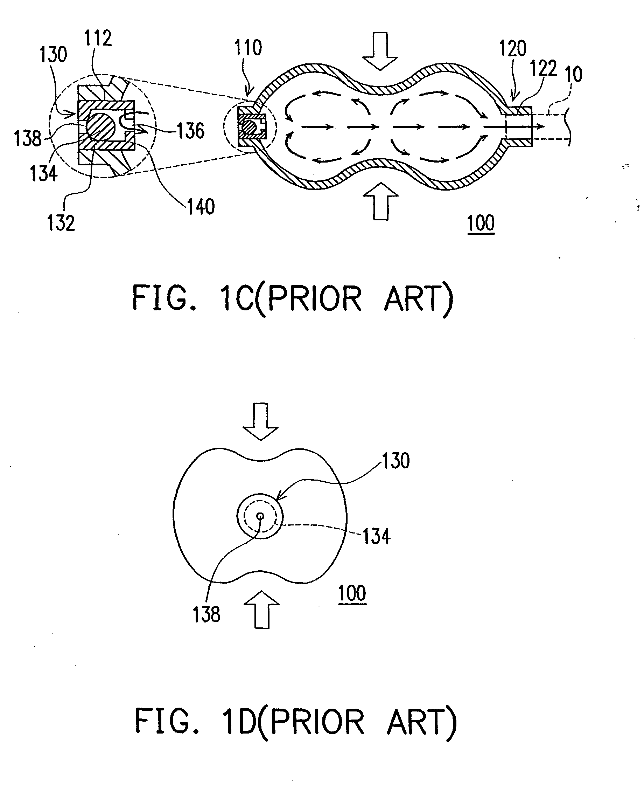

FIG. 1C and FIG. 1D are respectively longitudinal and transversal views illustrating a pressed state of the inflation bulb known in the prior art. When the inflation bulb 100 is pressed, the air gas therein flows toward the two ends of the inflation bulb 100. At the gas inlet end 110, the air gas enters the valve 130 and pushes the sealing ball 134 to occlude the through hole 138. The air gas therefore exits the inflation bulb 100 via the gas outlet opening 122. By repeated press and release of the inflation bulb 100, air gas is therefore delivered through the gas intake pipe 10 to inflate the cuff of the sphygmomanometer.

One disadvantage of the above structure is that the inflation bulb and the gas valve are distinct elements and therefore they have to be assembled with each other. This results in the increase of both fabrication cost and fabrication time. Furthermore, since the gas valve is usually made of a metallic material, the contact between the sealing ball and the through hole may be ineffective to achieve a hermetic sealing, which adversely lowers the yield of the inflation bulb.

According to the invention there is provided an inflation bulb, suitable for use in a sphygmomanometer, the inflation bulb being internally hollow and comprising a gas inlet end and a gas outlet end, wherein an inner side of the gas inlet end forms protruding lips that define a gas inlet opening through which exterior air gas is allowed to penetrate the inflation bulb, and the gas outlet end is provided with a gas outlet opening.

An aspect of the invention is therefore to provide an inflation bulb that includes a lip structure formed in a single body with the inflation bulb to replace the conventional gas valve.

The invention thus also provides an inflation bulb that is suitable for use with a sphygmomanometer, which may be a semi-automatic electric sphygmomanometer or a mercury type sphygmomanometer. The inflation bulb is internally hollow, and comprises a gas inlet end and a gas outlet end. An inner side of the gas inlet end forms inwardly protruding lips that may be approximately flat. The lips define a gas inlet opening through which exterior air gas is allowed to penetrate the inflation bulb, the lips being capable of occluding and opening the gas inlet opening. The gas outlet end includes a gas outlet opening. The gas outlet opening may be further connected to a gas intake pipe.

According to other embodiments of the invention, the gas inlet opening may include various shapes such as a bar opening, a cross-shaped opening, or a Y-shaped opening. When the inflation bulb is pressed, the lips occlude the gas inlet opening, which forces the air gas inside the inflation bulb to flow out through the gas outlet opening to the gas intake pipe. When the operator releases the pressing action, the inflation bulb recovers its initial state to have the lips open the gas inlet opening, and therefore air flows into the bulb. By repeated press and release of the inflation bulb, air gas is thereby delivered through the gas intake pipe to inflate a cuff of the sphygmomanometer.

According to a preferred embodiment of the invention, an outer wall of the gas inlet end is further provided with a reinforcement portion that is approximately annular around the lips. The reinforcement portion is formed preferably by increasing the thickness of the outer wall of the gas inlet end for locally increasing its hardness. The operator is thereby prevented from pressing the inflation bulb at the location of the gas inlet end. The reinforcement portion also enables it to prevent an inadequate deviation of the lips, which may disable the occlusion of the gas inlet opening.

It is to be understood that both the foregoing general description and the following detailed description are exemplary, and are intended to provide further explanation of the invention as claimed.

The accompanying drawings are included to provide a further understanding of the invention, and are incorporated in and constitute a part of this specification. The drawings illustrate embodiments of the invention and, together with the description, serve to explain the principles of the invention. In the drawings,
FIG. 1A and FIG. 1B are respectively longitudinal and transversal views illustrating an initial state of an inflation bulb for a sphygmomanometer known in the prior art;
FIG. 1C and FIG. 1D are respectively longitudinal and transversal views illustrating a pressed state of an inflation bulb for a sphygmomanometer known in the prior art;
FIG. 2A and FIG. 2B are respectively longitudinal and transversal views illustrating an initial state of an inflation bulb for a sphygmomanometer according to an embodiment of the invention;
FIG. 2C and FIG. 2D are respectively longitudinal and transversal views illustrating a pressed state of an inflation bulb for a sphygmomanometer according to an embodiment of the invention;
FIG. 2E and FIG. 2F are respectively longitudinal and transversal views illustrating a recovering state of an inflation bulb for a sphygmomanometer according to an embodiment of the invention; and
FIG. 3A and FIG. 3B are transversal views illustrating different variations of an inflation bulb according to the invention.

The following detailed description of the embodiments and examples of the present invention with reference to the accompanying drawings is only illustrative and not limiting. Furthermore, wherever possible in the description, the same reference symbols will refer to similar elements and parts unless otherwise illustrated in the drawings.

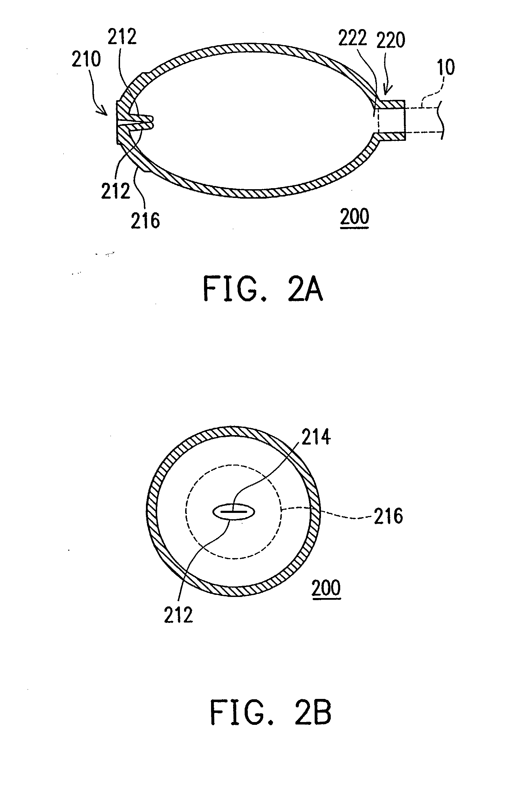

Reference now is made to FIG. 2A and FIG. 2B to describe an inflation bulb according to an embodiment of the invention. As illustrated, an inflation bulb 200 is inwardly hollow, and includes a gas inlet end 210 and a gas outlet end 220. An inner side of the gas inlet end 210 forms a pair of protruding lips 212 that are approximately flat longitudinally. The lips 212 form a gas inlet opening 214. The gas inlet opening 214 includes, but is not limited to, for example, a bar-shaped opening. In an initial state of the inflation bulb 200, the lips 212 occlude the gas inlet opening 214. The gas outlet end 220 is opened with a gas outlet opening 222 that is connected to a gas intake pipe 10. The gas intake pipe 10 is further connected to a gas reverse-flow preventing device (not shown), as it is conventionally known in the art. It should be noticed that the lips 212 are formed in a single body with the bulb 200. The lips 212 and the bulb 200 may be made of, for example but no limited to, flexible rubber. The gas inlet end 210 has an outer wall provided with a reinforcement portion 216. The reinforcement portion 216 appears generally annular around the lips 212.

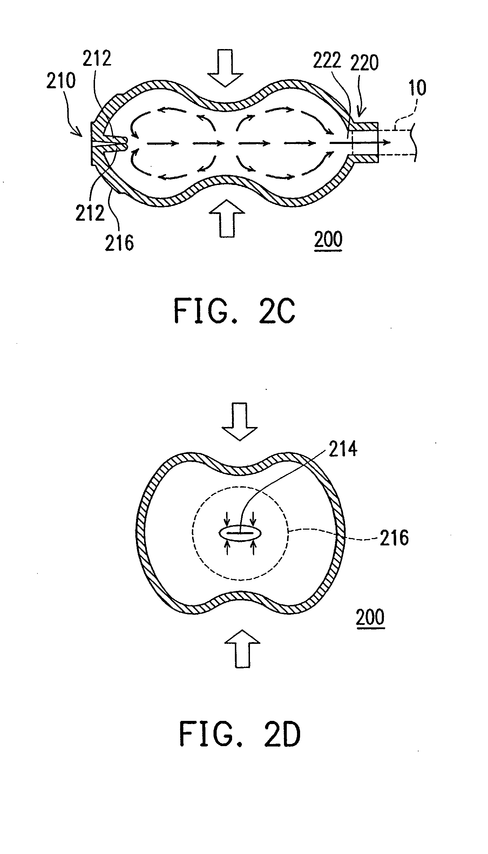

FIG. 2C and FIG. 2D are respectively longitudinal and transversal views illustrating a pressed state of the inflation bulb according to an embodiment of the invention.

When the inflation bulb 200 is pressed, the lips 212 occlude the gas inlet opening 214. Due to the pressing force, the air gas within the inflation bulb 200, blocked at the occluded gas inlet opening 214, is compelled to exit through the gas outlet end 220.

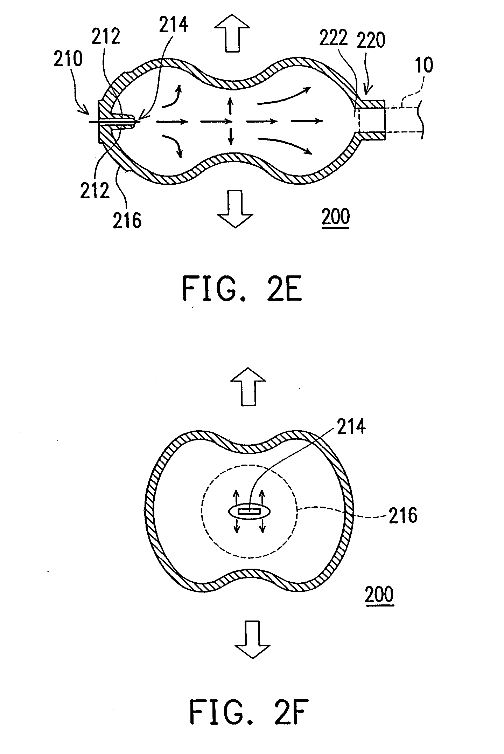

FIG. 2E and FIG. 2F are respectively longitudinal and transversal views illustrating a recovering state of the inflation bulb according to an embodiment of the invention.

By its own inherent characteristic, the inflation bulb 200 is capable of recovering its initial state when the operator releases the pressing action. While the inflation bulb 200 is recovering its initial state, the lips 212 open the gas inlet opening 214, which allows exterior air gas to penetrate the inflation bulb 200. Through the repeated press/release actions as described above, air gas is therefore progressively delivered through the gas intake pipe 10.

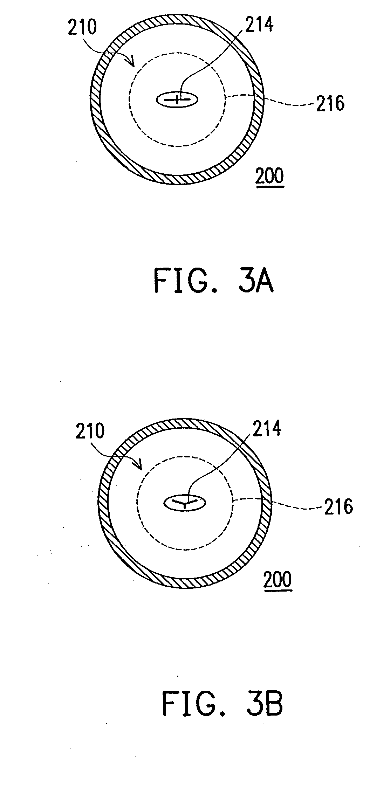

FIG. 3A and FIG. 3B are schematic views illustrating variant embodiments of the invention wherein the gas inlet opening 214 may include various adequate shapes such as a cross shape or a "Y" shape. Regardless of its shape, the gas inlet opening 214 is characterized in that it is opened when the inflation bulb 200 is recovering the initial configuration, and is occluded when the inflation bulb 200 is either in its initial configuration or pressed by the operator.

As described above, the reinforcement portion 216 is formed by, for example, increasing the thickness of the outer wall of the gas inlet end 210, which locally increases its hardness. The operator is thereby prevented from pressing the inflation bulb 200 at the location of the gas inlet end 210. The reinforcement portion 216 also prevents an inadequate deviation of the lips 212 from adversely occluding the gas inlet opening 214.

As described above, the invention includes at least the following advantages:-
(1) The lip structure and the inflation bulb are manufactured in one step, and the lip structure has the same function as the unidirectional gas valve of the prior art. The mount of the unidirectional gas valve therefore is unnecessary, and the fabrication cost and fabrication time are reduced; and
(2) The flexible rubber material enables an effective occlusion of the gas inlet opening without gas leakage, which improves the yield of the inflation bulb.

It should be apparent to those skilled in the art that other structures that are obtained from various modifications and variations of different parts of the above-described structures of the invention would be possible without departing from the scope and spirit of the invention as illustrated herein. Therefore, the above description of embodiments and examples only illustrates specific ways of making and performing the invention that, consequently, should cover variations and modifications thereof, provided they fall within the inventive concepts as defined in the following claims.

## Claims

1. An inflation bulb, suitable for use in a sphygmomanometer, the inflation bulb being internally hollow and comprising a gas inlet end and a gas outlet end, wherein an inner side of the gas inlet end forms protruding lips that define a gas inlet opening through which exterior air gas is allowed to penetrate the inflation bulb, and the gas outlet end is provided with a gas outlet opening.

2. An inflation bulb according to claim 1, **characterised in that** an outer wall of the gas inlet end further includes a reinforcement portion that increases the hardness of the gas inlet end.

3. An inflation bulb according to claim 2, **characterised in that** the reinforcement portion is formed in an annular shape around the lips.

4. An inflation bulb according to any preceding claim, **characterised in that** the gas inlet opening has a bar shape.

5. An inflation bulb according to any of claims 1 to 3, **characterised in that** the gas inlet opening has a cross shape.

6. An inflation bulb according to any of claims 1 to 3, **characterised in that** the gas inlet opening has a Y-shape.

7. An inflation bulb according to any preceding claim, **characterised in that** the gas outlet opening is further connected to a gas intake pipe.

8. An inflation bulb according to any preceding claim, **characterised in that** the lips are approximately flat.

9. An inflation bulb according to any preceding claim, **characterised in that** the lips are made of flexible rubber.

10. An inflation bulb according to any preceding claim, **characterised by** being further adapted with a sphygmomanometer selected from a group consisting of semi-automatic electric sphygmomanometer and a mercury type sphygmomanometer.
